# FASCICULE DE BREVET EUROPEEN

(11) **EP 2 066 408 B1**
(45) Date de publication et mention de la délivrance du brevet: **14.07.2010**
(21) Numéro de dépôt: 07823496.0
(22) Date de dépôt: 11.09.2007
(51) Int. Cl.: A61Q 19/06, A61K 8/35, A61K 8/97

(54) **COMPOSITION A BASE DE XANTHOXYLINE ET SON UTILISATION EN COSMETIQUE**
ZUSAMMENSETZUNG AUF XANTHOXYLINBASIS UND KOSMETISCHE VERWENDUNG DAFÜR
XANTHOXYLINE-BASED COMPOSITION AND COSMETIC USE THEREOF

(30) Priorité: 15.09.2006 FR 0608129
(43) Date de publication de la demande: 10.06.2009
(73) Titulaire: Pierre Fabre Dermo-Cosmétique, 92100 Boulogne (FR)
(72) Inventeur: CHARVERON, Marie, 31000 Toulouse (FR); BELAUBRE, Françoise, 31270 VILLENEUVE TOLOSANE (FR); BELLE, René, 81710 Saix (FR); DUNOUAU, Christophe, 31130 Pin-Balma (FR)
(74) Mandataire: Rousseau, Pierrick Edouard
(86) Numéro de dépôt international: PCT/FR2007/001467
(87) Numéro de publication internationale: WO 2008/031940

(56) Documents cités:
- WO-A-00/02570
- WO-A-01/76539
- WO-A-99/30699
- WO-A-2005/053720
- JP-A- 61 024 511
- SIMONSEN ET AL: "Antifungal constituents of Melicope borbonica" PHYTOTHERAPY RESEARCH, vol. 18, no. 7, 2004, pages 542-545, XP018000329
- DATABASE EMBASE [Online] ELSEVIER SCIENCE PUBLISHERS, AMSTERDAM, NL; 2002, LEE H Y ET AL: "Quality evaluation of Zanthoxyli Fructus" XP002430418 Database accession no. EMB-2002145868 & KOREAN JOURNAL OF PHARMACOGNOSY 2002 SOUTH KOREA, vol. 33, no. 1, 2002, pages 45-48, ISSN: 0253-3073
- DATABASE BIOSIS [Online] BIOSCIENCES INFORMATION SERVICE, PHILADELPHIA, PA, US; 1991, DE GODOY G F ET AL: "ANTIBACTERIAL ACTIVITY OF XANTHOXYLINE CONSTITUENT OF SEBASTIANIA-SCHOTTIANA" XP002430419 Database accession no. PREV199293014081 & FITOTERAPIA, vol. 62, no. 3, 1991, pages 269-270, ISSN: 0367-326X
- DATABASE BIOSIS [Online] BIOSCIENCES INFORMATION SERVICE, PHILADELPHIA, PA, US; 1990, CALIXTO J B ET AL: "ACTION OF 2 HYDROXY-4 6-DIMETHOXYACETOPHENONE ISOLATED FROM SEBASTIANIA-SCHOTTIANA" XP002430420 Database accession no. PREV199090007545 & PLANTA MEDICA, vol. 56, no. 1, 1990, pages 31-35, ISSN: 0032-0943

## Description

La présente invention a pour objet l'utilisation de la xanthoxyline, et des extraits de plantes la contenant pour le traitement de la cellulite.

La xanthoxyline définie comme le 4,6-dimethoxy-2-hydroxyacetophenone est également appelée la brevifoline. Cette molécule est d'origine naturelle. Elle est présente dans de nombreux végétaux regroupés principalement dans 2 familles botaniques, les Rutaceae où on la trouve présente dans les plantes suivantes = Mélicope borbonica, Phebalium tuberculosum, Phebalium filifolium, dans le genre Zanthoxylum avec Z. rhoifolium, Z.armatum, Z. bungeanum, Z. piperitum ; l'autre famille riche en xanthoxyline est celle des Euphorbiaceae avec Croton nepetaefolium, Hippomane mancinella, Sapium sebiferum, Sebastiana schottiana, Euphorbia quinquecostata et Euphorbia fidjiana. Elle est également décrite dans une Inuleae = Blumea balsamifera et une Asteraceae = Artemisia brevifolia. La xanthoxyline a déjà fait l'objet de certains travaux de valorisation.
Ses propriétés antifongique et antimicrobienne ont été largement travaillées. L'activité antifongique est modérée en particulier sur *Candida albicans* et *Penicillium expansum* (Simonsen H. T., Phytotherapy Research, Jul 2004, vol 18, n° 7, p 542-545) et sur les champignons pathogènes comme le *Trichophyton* (Pinheiro T.R. and coll. Arzneimittel - Forschung, Dec 1999, vol 49, n° 12, p 1039-1043). L'activité antibactérienne est également moyenne sur les bactéries Gram positives et Gram négatives (Gonzaga - Wellington A. and coll, Planta Medica, Apr 2003, vol 69, n° 4, p 371-374).
En thérapeutique, la xanthoxyline ainsi que certains dérivés furent travaillés essentiellement comme antispasmodique (Cechinel-Filho V. and coll, Journal of Pharmaceutical Sciences, Apr 1995, vol 84, n° 4, p 473-475; Calixto J. B. and coll, Planta Medica, Feb 1990, vol 56, n° 1, p 31-35 ; Hashimoto and coll, Planta Medica, 2001, vol 67 n° 2, p 179-81).
La xanthoxyline a également fait l'objet de protection dans le domaine thérapeutique comme modulateur des activités cystéine protéase (WO 9930699).
Dans le domaine cosmétique, le document JP-A-61024511 décrit une composition pour le traitement des lèvres gercées comprenant entre autres comme principe actif de la xanthoxyline (brevifoline). WO 0002570 concerne des extraits de *Zanthoxylum bungeanum* pouvant être obtenus par extraction du péricarpe avec du CO2 en conditions supercritiques et des compositions cosmétiques les contenant.

WO2005/053720 décrit des compositions topiques comprenant entre autres un extrait lipidique de *Zanthoxylum bungeanum* pour le traitement de la dermatite atopique, d'affections cutanées allergiques et de l'acné.
WO01/76539 traite enfin de compositions cosmétiques comprenant un extrait de *Zanthoxylum bungeanum* pour le traitement des cheveux.

De façon surprenante et inattendue, la demanderesse a mis en évidence une activité originale de la xanthoxyline, et d'extraits végétaux la contenant, sur l'inhibition de la différenciation des pré-adipocytes en adipocytes. Ce type d'activité est particulièrement intéressant dans l'application cosmétique amincissante, puisque lorsque les pré-adipocytes se différencient en adipocytes, cela se traduit par une surcharge de graisse encore plus importante au niveau du tissu adipeux et en particulier cellulitique.

La présente invention concerne donc l'utilisation de la xanthoxyline pour un traitement amincissant ; et pour la prévention ou le traitement des surcharges adipeuses et de la cellulite.

Au sens de la présente invention, la xanthoxyline peut être obtenue par voie chimique ou à partir d'un extrait végétal.

La présente invention concerne de préférence l'utilisation d'extraits végétaux contenant de la xanthoxyline : avantageusement, l'extrait végétal est issu des plantes de la famille des Rutaceae : Mélicope borbonica, Phebalium tuberculosum, Phebalium filifolium, dans le genre Zanthoxylum avec Z. rhoifolium, Z.armatum, Z. bungeanum, Z. piperitum ; des Euphorbiaceae : Croton nepetaefolium, Hippomane mancinella, Sapium sebiferum, Sebastiana schottiana, Euphorbia quinquecostata, Euphorbia fidjiana mais aussi de Blumea balsamifera et Artemisia brevifolia et autres plantes la contenant. Préférentiellement, la xanthoxyline est issue d'un extrait de Zanthoxylum bungeanum.
L'extrait végétal est préparé selon des étapes de préparation classiques connues de l'homme du métier.
La plante séchée préférentiellement est broyée puis extraite avec un solvant organique pouvant être un alcane (pentane, hexane, heptane, octane, cyclohexane), un éther oxyde (tetrahydrofurane, dioxane, diethyl éther), un ester (acétate d'éthyle, acétate d'isopropyle), un alcool (méthanol, éthanol, propanol, isopropanol, butanol), une cétone (méthyl éthyl cétone, dimethylcetone, méthyl isobutyl cétone), un hydrocarbure halogéné (chloroforme, dichlorométhane), l'eau et un mélange toute proportion miscible de ces solvants. L'extraction est réalisée dans un ratio plante/solvant compris entre environ 1/1 et environ 1/20 et peut être renouvelée 2 à 3 fois. La température du solvant d'extraction peut être égale à la température ambiante ou supérieure, pouvant atteindre la température d'ébullition du solvant engagé. Le temps de contact de la plante avec le solvant est compris entre environ 30 min et environ 72 heures.

On procède ensuite à une séparation solide/liquide, la plante étant séparée du solvant par filtration ou centrifugation.
Le filtrat obtenu peut être soit :
□ mis à sec directement par évaporation totale du solvant d'extraction et constituer l'extrait final,
□ conservé liquide dans le solvant d'extraction s'il est compatible avec son utilisation. Dans ce cas il peut être plus ou moins concentré par une étape d'évaporation,
□ purifié. Cette étape de purification peut être effectuée par des techniques connues de l'homme de l'art comme l'extraction liquide/liquide entre 2 solvants non miscibles, l'absorption sur un support comme de la silice, une résine échange d'ions, un support apolaire comme le polystyrène, la précipitation, la cristallisation, la sublimation. Après purification, l'extrait peut être séché par évaporation du solvant puis séchage, il peut être également mis en solution dans un solvant compatible avec son utilisation.

Un extrait obtenu par extraction, séparation solide/liquide puis séchage contient une fraction massique de xanthoxyline comprise entre 0,1 et 30 g pour 100 g de matière sèche de l'extrait, et préférentiellement comprise entre 1 et 15 g pour 100 g de matière sèche de l'extrait. Si l'extrait est maintenu en solution, la teneur en matière sèche de l'extrait liquide est comprise entre 0,1 g et 80 g pour 100 ml d'extrait liquide selon la concentration effectuée. La teneur en xanthoxyline peut s'exprimer selon la matière sèche contenue dans l'extrait liquide, elle sera alors comprise entre 0,1 et 30 g pour 100 g de cette matière sèche, et préférentiellement comprise entre 1 et 15 g pour 100 g de matière sèche.
Les techniques de purification permettent d'obtenir des extraits enrichis en xanthoxyline dont la fraction massique en xanthoxyline est supérieure à 30 g pour 100 g de matière sèche de l'extrait selon les techniques entreprises. Avantageusement, ladite fraction massique en xanthoxyline est comprise entre 50 g et 100 g pour 100 g de matière sèche de l'extrait enrichi.

La synthèse chimique de la xanthoxyline est connue de l'homme du métier. Elle est notamment décrite dans Hunan Huagong : 1999 : vol 29(6), P 27-28, ISSN :1005-8435.

La présente invention se rapporte également aux compositions cosmétiques comprenant de la xanthoxyline et au moins un autre principe actif amincissant. Au sens de la présente invention, la xanthoxyline agit comme principe actif pour la prévention ou le traitement des surcharges adipeuses et de la cellulite.
Un des aspects de la présente invention traite de nouvelles compositions cosmétiques amincissantes comprenant au moins de la xanthoxyline et au moins un autre principe actif amincissant en association avec un véhicule

Le véhicule cosmétique approprié peut être choisi entre autres parmi les agents diluants, les agents dispersants, les agents gélifiants, les gommes, les résines, les agents huileux, les alcools gras, les cires, les conservateurs, les colorants, les agents promoteurs d'absorption, les agents aromatisants ou parfumants, utilisés seuls ou en mélange.

Ladite composition cosmétique amincissante est de préférence sous forme topique ou orale.
Avantageusement, la forme topique est un gel, un spray, une crème, un gel crème, une pommade, un lait ou une lotion.
Cette composition peut aussi se présenter sous une forme orale, telle qu'un comprimé, gélule, poudre pour suspensions buvables.

De façon avantageuse, l'excipient cosmétique acceptable est approprié pour l'application de ladite composition par voie topique sur la zone de la peau à traiter ou pour l'application par voie orale de ladite composition.
De façon encore plus avantageuse, l'Homme du Métier adaptera un excipient cosmétique acceptable pour l'application de ladite composition par voie topique sur la zone de la peau à traiter. Le véhicule cosmétique est alors classiquement choisi par l'Homme du Métier afin de favoriser le passage de la xanthoxyline appliquée par voie cutanée, à travers la barrière cutanée et jusqu'aux adipocytes.
Le choix et/ou la quantité du ou des ingrédients dudit véhicule seront également déterminés par la tolérance et les besoins spécifiques de la peau sur laquelle la composition sera appliquée ainsi que par les propriétés et la consistance souhaitée pour la composition selon la présente invention.

L'efficacité amincissante de la composition selon la présente invention se traduit par :
- une perte centimétrique notamment au niveau des cuisses et/ou des hanches et/ou de la taille.
- une diminution de l'épaisseur du tissu adipeux, notamment au niveau des cuisses et/ou des hanches et/ou de la taille.
- et/ou par une diminution du volume des cuisses.

La quantité de xanthoxyline introduite dans la composition selon l'invention est comprise entre environ 0,1 mg et environ 100 mg pour 100 g de composition, préférentiellement entre environ 0,5 mg et environ 50 mg pour 100 g de composition, encore plus préférentiellement entre environ 1 mg et environ 20 mg.

La xanthoxyline desdites compositions peut être obtenue par voie chimique ou à partir d'un extrait végétal. De préférence, l'extrait est issu des plantes de la famille des Rutaceae : Mélicope borbonica, Phebalium tuberculosum, Phebalium filifolium, dans le genre Zanthoxylum avec Z. rhoifolium, Z.armatum, Z. bungeanum, Z. piperitum, des Euphorbiaceae : Croton nepetaefolium, Hippomane mancinella, Sapium sebiferum, Sebastiana schottiana, Euphorbia quinquecostata, Euphorbia fidjiana mais aussi de Blumea balsamifera et Artemisia brevifolia et autres plantes la contenant. De façon préférée, la xanthoxyline est extraite de Zanthoxylum bungeanum.

La présente invention concerne des compositions cosmétiques amincissantes à base de xanthoxyline comme principe actif amincissant, lesdites compositions comprennant en outre au moins un deuxième principe actif amincissant. Ce deuxième principe actif permet également de lutter contre les surcharges adipeuses et la cellulite.
Le ou les autres agents cosmétiques amincissants qui sont ajoutés à la présente composition sont connus de l'Homme du Métier, qui pourra ajuster les proportions relatives de chaque constituant de la composition afin d'optimiser l'efficacité de ladite composition.
Avantageusement, on citera à titre indicatif et non limitatif des principes actifs amincissants choisis dans le groupe formé par la caféine et ses sels, la phloridzine, la forskoline, l'hespéridine méthyl chalcone, le Guarana, le Maté, les extraits de Piloselle, de Ruscus, de Lierre, de branches de Pommier, de Coleus forskohlii, utilisés seuls ou en mélange.

Enfin, la présente invention concerne également une méthode de traitement cosmétique de la peau pour prévenir ou réduire les surcharges adipeuses et la cellulite qui consiste en une administration par voie topique sur la zone de la peau nécessitant un tel traitement ou par voie orale d'une composition à base de xanthoxyline. Avantageusement, la forme topique est choisie dans le groupe comprenant un gel, un spray, une crème, un gel crème, une pommade, un lait ou une lotion. Avantageusement, la forme orale est choisie dans le groupe comprenant des comprimés, des gélules et des poudres pour suspension buvable.

Les préparations et les compositions suivantes sont citées à titre d'exemples illustratifs et non limitatifs.

### EXEMPLES DE PREPARATION DE L'EXTRAIT VEGETAL

### EXEMPLE 1 : préparation d'un extrait de racines de Sebastiana schottiana.

5 kg de racines sèches broyées de Sebastiana schottiana sont extraites à 2 reprises par 30 1 et 20 1 d'éthanol à 95 % à reflux.
Après concentration des filtrats groupés à 30 1 environ on rajoute 101 d'alcool absolu, on glace à + 4 ° C une nuit et on filtre à froid rapidement sous pression pour éliminer les éventuelles cires présentes.
L'extrait fluide obtenu est dosé en hydroxy-methoxy-acétophénone.
Sa teneur en xanthoxyline se situe entre 0,1 et 5 % par rapport à la matière sèche.

### EXEMPLE 2 : préparation d'un extrait de péricarpes de fruits de Zanthoxylum bungeanum

100 kg de péricarpes de fruits de *Zanthoxylum bungeanum* sont broyés et extraits à reflux 2 fois avec 5001 d'heptane.
Après concentration en extrait mou on ajoute 10001 d'alcool absolu. On concentre jusqu'à élimination totale de l'heptane résiduel par azéotropie et on glace à + 4° pendant 1 nuit. Le floculat cireux est éliminé par filtration sous pression et l'extrait fluide est standardisé à 1 g/l en xanthoxyline.

### EXEMPLE 3 : préparation de xanthoxyline sous forme cristallisée à partir d'un extrait de péricarpes de fruits de Zanthoxylum bungeanum

100 kg de péricarpes de fruits de *Zanthoxylum bungeanum* broyés sont extraits à reflux par 5001 d'éthanol 90 %.
Le filtrat est concentré sous vide à 100 1 environ et le principe actif enrichi par extraction liquide/liquide par 3 x 201 d'héptane.
Les phases heptane groupées sont mises à sec. La xanthoxyline brute est redissoute dans 5 1 d'éthanol absolu chaud. Cette solution refroidie est soumise à un glaçage 24 h à + 2°C et filtrée froide très rapidement. Par concentration à environ 11 et repos à température ambiante on obtient environ 1 kg de xanthoxyline cristallisée, séparée par filtration et lavage sur filtre par 200 ml d'éthanol absolu froid, puis séchage sous vide à température ambiante. Sa pureté se situe entre 95 et 100 %.

### EXEMPLE 4 : préparation de xanthoxyline sous forme cristallisée à partir d'un extrait de péricarpes de fruits de Zanthoxylum bungeanum

Il est également possible d'obtenir le principe actif par sublimation directe à partir des péricarpes broyés chauffés au bain-marie bouillant et en opérant sous un vide de 15 mm Hg. La xanthoxyline est recueillie cristallisées sur le condenseur froid (10° C), sa pureté se situe entre 95 et 100 %.

### EXEMPLE DE COMPOSITION COSMETIQUE

### Gel crème amincissant n°1:

- Xanthoxyline 0,001 à 1%
- Hespéridine méthyl chalcone 0,3 à 1 %
- Caféine base 1 à 5%
- Acide caféique carboxylique 2 %
- Extrait de branches de pommiers 0,1 à 5 %
- Triglycérides caprique caprylique 20%
- Ethanol 95% 10%
- Triéthanolamine 0,3%
- Polymère carboxyvinylique 0,3%
- Parfum q.s.
- Conservateurs colorants
- Eau potable q.s.p. 100g

### Gel crème amincissant n°2:

- Xanthoxyline 0,001 à 0,02 %
- Hespéridine méthyl chalcone 0,1 à 5 %
- Caféine base 1 à 5%
- Acide caféique carboxylique 0,1 à 5 %
- Extrait de branches de pommiers 0,1 à 5 %
- Triglycérides caprique caprylique 15 à 25 %
- Ethanol 95% 10%
- Triéthanolamine 0,3%
- Polymère carboxyvinylique 0,3%
- Parfum q.s.
- Conservateurs colorants
- Eau potable q.s.p. 100g

### Spray amincissant zones rebelles :

- Extrait de Zanthoxylum bungeanum 1 à 5%
- Acide caféique carboxylique 5%
- Extraits de branches de pommier 0,1 à 10%
- Carbonate de guanidine 0,5%
- Extrait fluide de Ruscus 1%
- Alcool polyvinylique 0,2%
- P.E.G. 400 1%
- Alcool à 95% 30%
- Eau potable q.s.p. 100g

### Crème de massage amincissante décongestionnante :

- Extrait de Zanthoxylum bungeanum 1 à 10%
- Extraits de branches de pommiers 0,1 à 10%
- Extrait de piloselle 1 à 5%
- Caféine carboxylate de 3 nicotinol 1 à 20%
- Acétate de vitamine E 0,5%
- Excipient de massage q.s.p. 100g

### EVALUATION PHARMACOLOGIQUE

### ETUDE IN VITRO DE L'ACTION D'UN EXTRAIT SEC DE ZANTHOXYLUM BUNGEANUM ET DE XANTHOXYLINE SUR LA DIFFERENCIATION ADIPOCYTAIRE

La différenciation adipocytaire (différenciation des pré-adipocytes en adipocytes) est un phénomène biologique complexe régulé au niveau moléculaire par l'activation de gènes spécifiques conduisant à un phénotype adipocytaire particulier caractérisé par l'accumulation de gouttelettes lipidiques.
Les adipocytes sont capables d'hydrolyser les triglycérides et de libérer ainsi des acides gras et du glycérol, traceur de la lipolyse.
La demanderesse a étudié d'une part l'effet d'un extrait sec de Zanthoxylum bungeanum, et d'autre part l'effet de la xanthoxyline sur le processus de différenciation et prolifération adipocytaires. La référence utilisée pour cette étude est la cytokine TNFα (connue pour inhiber l'accumulation des triglycérides) dans le modèle de différenciation des adipocytes durant une longue période de culture (Journal of Clinical Endocrinology and Metabolism : PETRUSCHKE T., 1993, 76(3) :742-747).

### Protocole expérimental :

Les pré-adipocytes 3T3F442A sont capables de se différencier en adipocytes en présence d'insuline, de 3-isobutyl-1-méthylxanthine et de dexaméthasone (the Journal of Pharmacology and Expérimental Therapeutics : RIVAL Y., 2004, 311(2) : 467-475). Cette différenciation s'accompagne d'une accumulation de gouttelettes intracellulaires de triglycérides mises en évidence par le réactif Adipored Assay Reagent (CAMBREX, PT-7009).

Les cellules 3T3F442A sont cultivées dans un milieu DMEM (GIBCOBRL, ref 32430-027) contenant 10% de sérum de veau fatal à 37°C, 5% CO2 en atmosphère humide. Elles sont ensemencées à 5000 cellules par puits (plaque 96 puits). Deux jours après l'obtention d'un tapis cellulaire confluent, les cellules sont mises en condition de différenciation dans du milieu contenant 1,7 µM d'insuline, 0,5M de 3-isobutyl-1-méthylxanthine et 1 µM de dexaméthasone, en présence ou pas des produits pendant 7 jours. Les produits testés sont d'une part la forme sèche de l'extrait alcoolique de Zanthoxylum bungeanum titré à 1 g/l de xanthoxyline préparé selon l'exemple 2 comparativement au TNFα humain (R&D, 210-TA) ; et d'autre part de la xanthoxyline obtenue selon l'exemple 3 comparativement au TNFα humain (R&D, 210-TA).

La visualisation et la quantification des vacuoles lipidiques intracellulaires au niveau des cellules différenciées sont analysées à l'aide du réactif Adipored, colorant fluorescent spécifique des triglycérides (lipides neutres et amphiphiles) (Journal of Cell Biology : GREENSPAN P., 1985, 100 :965-973).
Le milieu de culture est enlevé et les cellules sont rincées au tampon phosphate D-PBS (GIBCOBRL, ref 21300-058). Le réactif Adipored est alors ajouté. Après 10 minutes d'incubation à température ambiante, la fluorescence est mesurée (excitation 485 nm, émission 530 nm). Une analyse histologique au microscope confocal Laser (ZEISS LSM 410 Invert Laser scan Microscope) est également réalisée à 543 nm.

### Résultats :

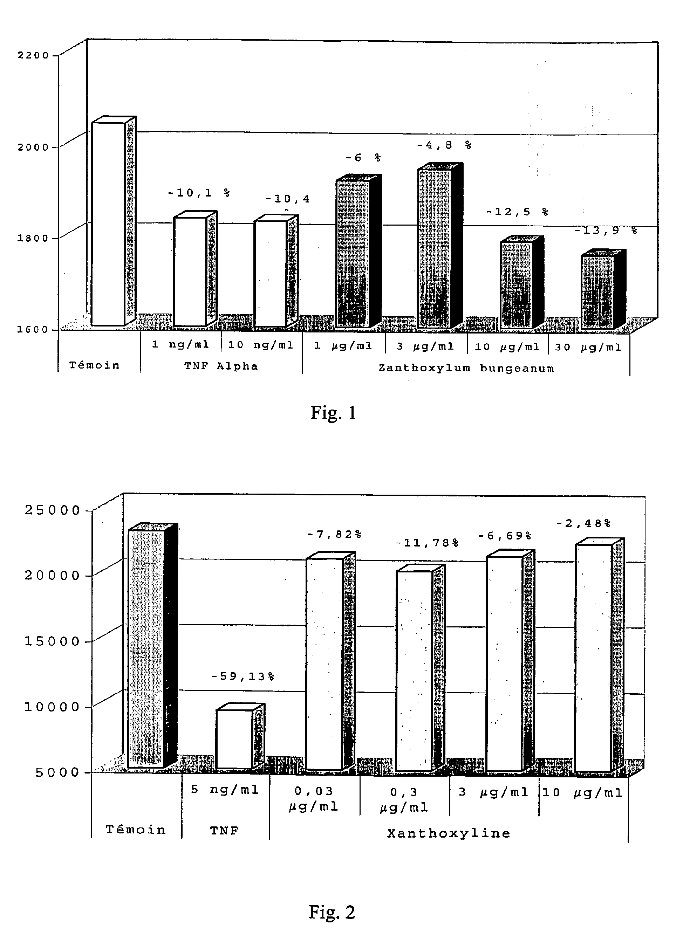

Les figures 1 et 2 représentent l'accumulation des triglycérides dans les cellules différenciées (exprimées en unité arbitraire d'Adipored) lorsque la différenciation a été induite respectivement par différentes concentrations en extrait de Zanthoxylum bungeanum et en xanthoxyline.

Sept jours après le traitement par un mélange insuline, 3-isobutyl-1-methylxanthine, dexaméthasone, les pré-adipocytes 3T3F442A se différencient en adipocytes. Leur morphologie change, les cellules s'arrondissent et accumulent des triglycérides visualisés par le colorant Adipored au microscope confocal.
La différenciation de la lignée cellulaire a été induite en présence des différents produits à tester et a été estimée par quantification de l'accumulation des triglycérides.

### - extrait de Zanthoxylum bungeanum :

Les résultats sont présentés sur la figure 1 : la première mesure correspond au témoin différencié en l'absence de produit à tester , puis les deux séries de mesure représentées correspondent respectivement au TNFα à 1 et 10 ng/ml et à l'extrait de Zanthoxylum bungeanum à 1, 3, 10 et 30 µg/ml.

On observe que l'extrait de Zanthoxylum bungeanum inhibe l'accumulation de triglycérides de façon significative à 10 et 30 µg/ml.

### - xanthoxyline :

Les résultats sont présentés sur la figure 2 : la première mesure correspond au témoin différencié en l'absence de produit à tester , la deuxième mesure correspond au TNFα à 5 ng/ml et enfin la série de mesures suivante correspond respectivement à des concentrations en xanthoxyline de 10, 3, 0,3 et 0,03 µg/ml.
On observe que la xanthoxyline présente une activité optimale (12% d'inhibition) à la concentration de 0,3 µg/mL

### ETUDE D'EFFICACITE D'UNE COMPOSITION A BASE DE XANTHOXYLINE

L'étude clinique présentée ci-après a été réalisée en ouvert, auprès de 61 femmes, âgées de 25 à 45 ans ayant un Indice de Masse Corporelle compris entre 22 et 26 kg.m⁻².

Cette étude concerne la composition gel crème n°2 donnée précédemment en exemple.
Le produit a été appliqué une fois par jour le matin pendant 28 jours sur les hanches, les fesses ; le ventre et une cuisse selon randomisation. A J28, le panel était de 60 femmes.

On note J0 le point de départ de cette étude d'efficacité (avant la 1^{ère} application).

Différentes techniques d'évaluation ont été utilisées : des mesures centimétriques, des mesures d'épaisseur du tissu adipeux et des mesures du volume des cuisses.

Les figures introduites ci-après représentent :
- figure 3 : évolution de l'épaisseur (mm) du tissu adipeux des cuisses sur l'ensemble du panel
- figures 4 et 5 : plans de référence horizontaux déterminant le volume mesuré des cuisses
- figure 6 : évolution (%) du volume des cuisses sur l'ensemble du panel.

### 1. Mesure centimétriques

### - Protocole :

Les mesures centimétriques sont réalisées au niveau :
   - des 2 cuisses
   - de la taille (niveau du nombril)
   - des hanches
Les mesures centimétriques sont réalisées après un repérage de chaque site en utilisant un rail vertical gradué équipé d'un laser qui détermine la hauteur par rapport au sol et permet un positionnement vertical correct.
Après un marquage au crayon de 4 traits horizontaux sur la circonférence de chaque site, la mesure est obtenue au moyen d'un mètre souple, appliqué précisément et sans compression sous cette ligne.

### - Résultats :

Les résultats centimétriques sur les différentes zones sont regroupés dans le tableau 1 pour les résultats à J14 (après 14 jours de traitement) et à J28 (après 28 jours de traitement).

Sont relevés :
- tour de cuisse = tour de cuisse traitée à J14 ou J28 - tour de cuisse à J0
   La moyenne de ces valeurs pour l'ensemble des sujets de l'étude est précisée ainsi que la valeur maximale obtenue.
- différence entre le tour de cuisse traitée et le tour de cuisse non traitée à J14 ou J28 La différence maximale entre la cuisse traitée et la cuisse non traitée est précisée.
- tour des hanches = tour des hanches traitées à J14 ou J28 - tour des hanches à J0 La moyenne de ces valeurs pour l'ensemble des sujets de l'étude est précisée ainsi que la valeur maximale obtenue.
- Tour de taille = tour de taille traitée à J14 ou J28 - tour de taille à J0.
   La moyenne de ces valeurs pour l'ensemble des sujets de l'étude est précisée ainsi que la valeur maximale obtenue.

**Tableau 1 : résultats centimétriques à J14 et J28**

| | Composition selon la présente invention | |
|---|---|---|
| | **J14** | **J28** |
| *Mesures en cm* | Moyenne | Moyenne |
| | (n=61) | (n=60) |
| **Tour de cuisse** | -0.5 | -0,8 |
| Max | -1,5 | -2,2 |
| | | |
| (Tour de cuisse traitée) - (tour de cuisse non traitée) | -0,3 | -0,6 |
| Max | -1,1 | -1,8 |
| **Tour de Hanches** | -0,7 | -1,0 |
| Max | -3,3 | -3,8 |
| **Tour de Taille** | -0,6 | -0,8 |
| Max | -2.9 | -2,5 |

### 2. Mesures de l'épaisseur du tissu adipeux des cuisses (par échographie)

### - Protocole :

Le matériel utilisé est un échographe type EUB 415 - HITACHI - JAPON équipé d'une sonde linéaire opérant à une fréquence de 7,5 MHz.

L'imagerie ultrasonore consiste en la réalisation d'une cartographie des différences d'impédances des tissus concernés vis-à-vis des ondes ultrasonores. Après émission d'un faisceau d'ondes par une sonde, la réception des échos et la valeur de leur intensité liée à leur absorption par les différents tissus, permettent de construire une image dans le plan du faisceau.
Trois images sont acquises successivement au niveau du repère cutané de chaque site de mesure. Sur chaque cliché, trois mesures d'épaisseur sont effectuées.
Les mesures sont réalisées au niveau du sommet du renflement cellulitique situé sur la face externe de chaque cuisse.
La valeur retenue correspond à la moyenne des 3 mesures effectuées.

### - Résultats :

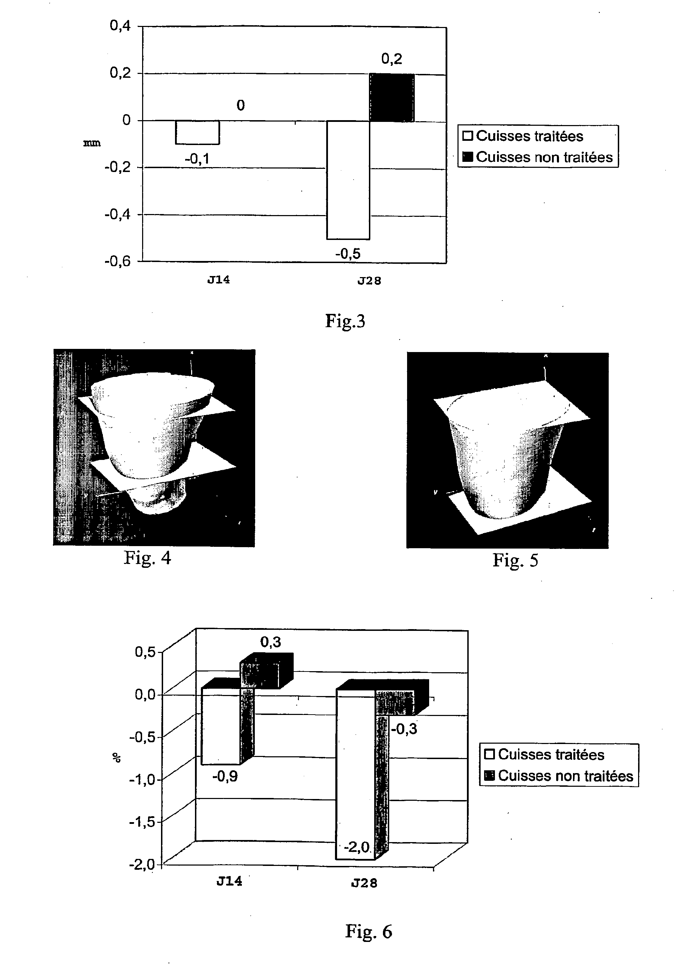

Les résultats après 14 et 28 jours de traitement (respectivement notés J14 et J28) sont présentés sur la figure 3 : ces résultats expriment la différence (en mm) entre l'épaisseur du tissu adipeux à J14 ou J28 et l'épaisseur du tissu adipeux de la même cuisse à J0.
Les mesures obtenues sur les cuisses traitées (□) ont été comparées avec les mêmes mesures sur les cuisses non traitées (■).
Après 14 jours de traitement: une diminution moyenne de l'épaisseur du tissu adipeux de 0,1 mm est constatée sur n= 29 sujets.
Après 28 jours de traitement : une diminution significative moyenne de l'épaisseur du tissu adipeux de 0,5 mm est constatée, soit une réduction de 1,6% l'épaisseur du tissu adipeux sur n= 26 sujets.

### 3. Mesures du volume des cuisses (technique de projection de franges « in vivo »)

### - Protocole :

La technique des projections de franges, basée sur le principe de la triangulation optique avec une lumière structurée, permet des acquisitions en trois dimensions du volume de chaque cuisse. Elle permet de visualiser et de quantifier la variation du volume des cuisses.
Ce système comprend un capteur de mesure associant un projecteur halogène couplé à une caméra CCD haute résolution 768 x 576 pixels, calibrée pour un champ de mesure de 240 mm - système MicroTop (EoTech, France) - interfacé au logiciel d'acquisition Optocat (EoTech, France). La résolution moyenne est d'environ 150 µm dans les 3 directions de l'espace (x, y, z).
Ce système permet de projeter un réseau de lignes sur la zone à mesurer, les déformations de celles-ci sont enregistrées par la caméra en vue d'un traitement informatique.
Une série de 6 acquisitions est réalisée à différentes incidences (0°, 60°, 120°, 180°, 240°, et 300°) par rapport au centre de rotation de la zone à mesurer. Les 6 acquisitions sont recalées automatiquement par réalignement géométrique, permettant la reconstruction de la surface de la cuisse. Le volume est déterminé après définition de 2 plans de référence horizontaux (figures 4 et 5).
Chaque plan est défini après superposition des reconstructions 3D d'une cuisse donnée, aux différents temps de la cinétique afin d'obtenir un repositionnement reproductible.
Ces plans horizontaux déterminent le volume mesuré des cuisses.

### - Résultats :

Les résultats après 14 et 28 jours de traitement (notés J14 et J28) sont présentés sur la figure 6 : ils expriment l'évolution (en %) du volume de la cuisse à J14 ou J28 par rapport au volume de cette même cuisse à J0.

Les mesures obtenues sur les cuisses traitées (□) ont été comparées avec les mêmes mesures sur les cuisses non traitées (■).
Après 14 jours de traitement: on observe une diminution significative du volume des cuisses traitées de 0,9% par rapport à J0 sur n = 29 sujets. La différence (cuisse traitée - cuisse non traitée) est également significative.
Après 28 jours de traitement: on observe une diminution significative du volume des cuisses traitées de 2% par rapport à J0 sur n= 27 sujets. La différence (cuisse traitée - cuisse non traitée) est également significative.

### CONCLUSION DE L'ETUDE D'EFFICACITE

Cette étude a permis de démontrer l'efficacité amincissante d'une composition selon la présente invention.
Les pourcentages de sujets répondants sont très importants. Au bout de 28 jours, 95% des sujets présentent une perte centimétrique au niveau de la cuisse traitée par rapport à J0; de même pour 90% des sujets au niveau des hanches.
Dès 14 jours, les différences au niveau de la cuisse traitée, des hanches et de la taille par rapport à J0 sont significatives. A 28 jours ces résultats s'accentuent.
La différence (cuisse traitée - cuisse non traitée), qui est plus représentative de l'efficacité, est, elle aussi, significative dès 14 jours.
Les résultats significatifs de l'échographie et de la projection de franges confirment les résultats obtenus par la centimétrie.

## Revendications

1. Utilisation de la xanthoxyline pour un traitement amincissant.

2. Utilisation de la xanthoxyline pour la prévention ou le traitement des surcharges adipeuses et de la cellulite.

3. Utilisation selon la revendication 1 ou 2 **caractérisée en ce que** la xanthoxyline est obtenue à partir d'un extrait végétal.

4. Utilisation selon la revendication 3, **caractérisée en ce que** l'extrait végétal est issu des plantes de la famille des Rutaceae : Mélicope borbonica, Phebalium tuberculosum, Phebalium filifolium, dans le genre Zanthoxylum avec Z. rhoifolium, Z.armatum, Z. bungeanum, Z. piperitum ; des Euphorbiaceae : Croton nepetaefolium, Hippomane mancinella, Sapium sebiferum, Sebastiana schottiana, Euphorbia quinquecostata, Euphorbia fidjiana et de Blumea balsamifera et Artemisia brevifolia.

5. Utilisation selon la revendication 3, **caractérisée en ce que** l'extrait végétal est issu de Zanthoxylum bungeanum.

6. Utilisation selon l'une quelconque des revendications 3 à 5 **caractérisée en ce que** la fraction massique de xanthoxyline dans ledit extrait est comprise entre 0,1 et 30 g pour 100 g d'extrait sec.

7. Utilisation selon l'une quelconque des revendications 3 à 6 **caractérisée en ce que** l'extrait végétal est enrichi en xanthoxyline, ledit extrait enrichi contenant une fraction massique de xanthoxyline supérieure à 30 g pour 100 g d'extrait sec.

8. Composition cosmétique amincissante comprenant de la xanthoxyline et au moins un autre principe actif amincissant en association avec un véhicule cosmétique approprié.

9. Composition cosmétique amincissante selon la revendication 8 **caractérisée en ce que** les autres principes actifs amincissants sont choisis dans le groupe formé par la caféine et ses sels, la phloridzine, la forskoline, l'hespéridine méthyl chalcone, le Guarana, le Maté, les extraits de Piloselle, de Ruscus, de Lierre, de branches de pommiers et de Coleus forskohlii, utilisés seuls ou en mélange.

10. Composition cosmétique amincissante selon l'une quelconque des revendications 8 à 9 **caractérisée en ce que** la xanthoxyline est obtenue à partir d'un extrait végétal.

11. Composition cosmétique amincissante selon la revendication 10 **caractérisée en ce que** l'extrait végétal est issu des plantes de la famille des Rutaceae : Mélicope borbonica, Phebalium tuberculosum, Phebalium filifolium, dans le genre Zanthoxylum avec Z. rhoifolium, Z.armatum, Z. bungeanum, Z. piperitum ; des Euphorbiaceae : Croton nepetaefolium, Hippomane mancinella, Sapium sebiferum, Sebastiana schottiana, Euphorbia quinquecostata, Euphorbia fidjiana et de Blumea balsamifera et Artemisia brevifolia.

12. Composition cosmétique amincissante selon la revendication 10 **caractérisée en ce que** l'extrait végétal est issu de Zanthoxylum bungeanum.

13. Composition cosmétique amincissante selon l'une quelconque des revendications 8 à 12 **caractérisée en ce qu'**elle se présente sous forme orale ou sous forme topique.

14. Composition cosmétique amincissante selon la revendication 13 **caractérisée en ce que** la forme topique est choisie parmi le groupe comprenant un gel, un spray, une crème, un gel crème, une pommade, un lait ou une lotion.

15. Composition cosmétique amincissante selon la revendication 13 **caractérisée en ce que** la forme orale est choisie parmi le groupe comprenant des comprimés, des gélules et des poudres pour suspensions buvables.

16. Méthode de traitement cosmétique de la peau pour prévenir ou réduire les surcharges adipeuses et la cellulite **caractérisée en ce qu'**elle consiste en une administration par voie topique sur la zone de la peau nécessitant un tel traitement ou par voie orale de xanthoxyline.

17. Méthode selon la revendication 16 **caractérisée en ce que** la forme topique est choisie parmi un gel, un spray, une crème, un gel crème, une pommade, un lait ou une lotion.

18. Méthode selon la revendication 16 **caractérisée en ce que** la forme orale est choisie parmi des comprimés, des gélules et des poudres pour suspensions buvables.

## Claims

1. Use of xanthoxyline for a slimming treatment.

2. Use of xanthoxyline for the prevention or treatment of excess adipose and cellulite.

3. Use according to claim 1 or 2, **characterised in that** xanthoxyline is obtained from a plant extract.

4. Use according to claim 3, **characterised in that** the plant extract originates from plants from the family of the Rutaceae: Melicope borbonica, Phebalium tuberculosum, Phebalium filifolium, and in the genus Zanthoxylum with Z. rhoifolium, Z. armatum, Z. bungeanum, Z. piperitum ; the Euphorbiaceae : Croton nepetaefolium, Hippomane mancinella, Sapium sebiferum, Sebastiana schottiana, Euphorbia quinquecostata, Euphorbia fidjiana and from Blumea balsamifera and Artemisia brevifolia.

5. Use according to claim 3, **characterised in that** the plant extract originates from Zanthoxylum bungeanum.

6. Use according to any one of claims 3 to 5, **characterised in that** the fraction by weight of xanthoxyline in the said extract is between 0.1 and 30 g inclusive per 100 g of dry extract.

7. Use according to any one of claims 3 to 6, **characterised in that** the plant extract is enriched with xanthoxyline, the said enriched extract containing a fraction by weight of xanthoxyline greater than 30 g per 100 g of dry extract.

8. Cosmetic slimming composition comprising xanthoxyline and at least one other slimming active ingredient in association with a suitable cosmetic carrier.

9. Cosmetic slimming composition according to claim 8, **characterised in that** the other slimming active ingredients are selected from the group formed by caffeine and its salts, phloridzin, forskolin, hesperidin methyl chalcone, guarana, maté, extracts of mouse-ear hawkweed, of ruscus, of ivy, of apple tree branches and of Coleus forskohlii, used alone or in a mixture.

10. Cosmetic slimming composition according to any one of claims 8 to 9, **characterised in that** xanthoxyline is obtained from a plant extract.

11. Cosmetic slimming composition according to claim 10, **characterised in that** the plant extract originates from plants from the family of the Rutaceae : Melicope borbonica, Phebalium tuberculosum, Phebalium filifolium, and in the genus Zanthoxylum with Z. rhoifolium, Z. armatum, Z. bungeanum, Z. piperitum; the Euphorbiaceae: Croton nepetaefolium, Hippomane mancinella, Sapium sebiferum, Sebastiana schottiana, Euphorbia quinquecostata, Euphorbia fidjiana and from Blumea balsamifera and Artemisia brevifolia.

12. Cosmetic slimming composition according to claim 10, **characterised in that** the plant extract originates from Zanthoxylum bungeanum.

13. Cosmetic slimming composition according to any one of claims 8 to 12, **characterised in that** it is in oral form or in topical form.

14. Cosmetic slimming composition according to claim 13, **characterised in that** the topical form is selected from the group consisting of a gel, a spray, a cream, a cream gel, an ointment, a milk or a lotion.

15. Cosmetic slimming composition according to claim 13, **characterised in that** the oral form is selected from the group consisting of tablets, capsules and powders for drinkable suspensions.

16. Method of cosmetic treatment of the skin to prevent or reduce excess adipose and cellulite, **characterised in that** it comprises the administration of xanthoxyline topically, to the zone of the skin requiring such treatment, or orally.

17. Method according to claim 16, **characterised in that** the topical form is selected from a gel, a spray, a cream, a cream gel, an ointment, a milk or a lotion.

18. Method according to claim 16, **characterised in that** the oral form is selected from tablets, capsules and powders for drinkable suspensions.

## Patentansprüche

1. Verwendung von Xanthoxylin für eine Behandlung zur Verringerung des Körperumfangs.

2. Verwendung von Xanthoxylin für die Vorbeugung oder die Behandlung von körperfettbedingtem Übergewicht und Zellulitis.

3. Verwendung nach Anspruch 1 oder 2, **dadurch gekennzeichnet, dass** das Xanthoxylin aus einem Pflanzenextrakt gewonnen wird.

4. Verwendung nach Anspruch 3, **dadurch gekennzeichnet, dass** der Pflanzenextrakt von Pflanzen der Familie der Rautengewächse stammt: Mélicope borbonica, Phebalium tuberculosum, Phebalium filifolium, in der Gattung Zanthoxylum mit Z. rhoifolium, Z. armatum, Z. bungeanum, Z. piperitum; Wolfsmilchgewächse: Croton nepetaefolium, Hippomane mancinella, Sapium sebiferum, Sebastiana schottiana, Euphorbia quinquecostata, Euphorbia fidjiana und von Blumea balsamifera und Artemisia brevifolia.

5. Verwendung nach Anspruch 3, **dadurch gekennzeichnet, dass** der Pflanzenextrakt von Zanthoxylum bungeanum stammt.

6. Verwendung nach einem beliebigen der Ansprüche 3 bis 5, **dadurch gekennzeichnet, dass** der Massenanteil des Xanthoxylins in dem Extrakt 0,1 bis 30 g auf 100 g Trockenmasse beträgt.

7. Verwendung nach einem beliebigen der Ansprüche 3 bis 6, **dadurch gekennzeichnet, dass** der Pflanzenextrakt mit Xanthoxylin angereichert ist, wobei der angereicherte Extrakt einen Massenanteil an Xanthoxylin von mehr als 30 g auf 100 g Trockenmasse enthält.

8. Kosmetische Zusammensetzung zur Verringerung des Körperumfangs, die Xanthoxylin und mindestens einen weiteren Wirkstoff zur Verringerung des Körperumfangs in Verbindung mit einem geeigneten kosmetischen Trägerstoff umfasst.

9. Kosmetische Zusammensetzung zur Verringerung des Körperumfangs nach Anspruch 8, **dadurch gekennzeichnet, dass** die weiteren Wirkstoffe zur Verringerung des Körperumfangs aus der Gruppe gewählt sind, die von Coffein und seinen Salzen, Phloridzin, Forskolin, Hesperidin-Methylchalkon, Guarana, Mate, Extrakten des kleinen Habichtskrauts, des Mäusedorns, des Efeus, von Apfelbaumzweigen und von Coleus forskohlii, einzeln oder in Mischung verwendet, gebildet wird.

10. Kosmetische Zusammensetzung zur Verringerung des Körperumfangs nach einem beliebigen der Ansprüche 8 bis 9, **dadurch gekennzeichnet, dass** das Xanthoxylin aus einem Pflanzenextrakt gewonnen wird.

11. Kosmetische Zusammensetzung zur Verringerung des Körperumfangs nach Anspruch 10, **dadurch gekennzeichnet, dass** der Pflanzenextrakt von Pflanzen der Familie der Rautengewächse stammt: Mélicope borbonica, Phebalium tuberculosum, Phebalium filifolium, in der Gattung Zanthoxylum mit Z. rhoifolium, Z. armatum, Z. bungeanum, Z. piperitum; Wolfsmilchgewächse: Croton nepetaefolium, Hippomane mancinella, Sapium sebiferum, Sebastiana schottiana, Euphorbia quinquecostata, Euphorbia fidjiana und von Blumea balsamifera und Artemisia brevifolia.

12. Kosmetische Zusammensetzung zur Verringerung des Körperumfangs nach Anspruch 10, **dadurch gekennzeichnet, dass** der Pflanzenextrakt von Zanthoxylum bungeanum stammt.

13. Kosmetische Zusammensetzung zur Verringerung des Körperumfangs nach einem beliebigen der Ansprüche 8 bis 12, **dadurch gekennzeichnet, dass** sie in oraler Form oder in topischer Form vorliegt.

14. Kosmetische Zusammensetzung zur Verringerung des Körperumfangs nach Anspruch 13, **dadurch gekennzeichnet, dass** die topische Form aus der Gruppe gewählt ist, die ein Gel, ein Spray, eine Creme, eine gelartige Creme, eine Salbe, eine Milch oder eine Lotion umfasst.

15. Kosmetische Zusammensetzung zur Verringerung des Körperumfangs nach Anspruch 13, **dadurch gekennzeichnet, dass** die orale Form aus der Gruppe gewählt ist, die Tabletten, Gelkapseln und Pulver für trinkfähige Suspensionen umfasst.

16. Verfahren zur kosmetischen Behandlung der Haut, um körperfettbedingtem Übergewicht und Zellulitis vorzubeugen oder diese zu behandeln, **dadurch gekennzeichnet, dass** es darin besteht, dass Xanthoxylin auf topischem Wege auf dem Hautbereich, der eine derartige Behandlung benötigt, oder auf oralem Wege verabreicht wird.

17. Verfahren nach Anspruch 16, **dadurch gekennzeichnet, dass** die topische Form aus einem Gel, einem Spray, einer Creme, einer gelartigen Creme, einer Salbe, einer Milch oder einer Lotion gewählt ist.

18. Verfahren nach Anspruch 16, **dadurch gekennzeichnet, dass** die orale Form aus Tabletten, Gelkapseln und Pulvern für trinkfähige Suspensionen gewählt ist.
